# EUROPÄISCHE PATENTANMELDUNG

(11) **EP 2 119 427 A2**
(43) Veröffentlichungstag der Anmeldung: **18.11.2009**
(21) Anmeldenummer: 09005805.8
(22) Anmeldetag: 27.04.2009
(51) Int. Cl.: A61K 8/06, A61K 8/81, A61K 8/97, A61Q 19/00

(54) **Viskositätsregulierte kosmetische Zubereitung**

(30) Priorität: 14.05.2008 DE 102008001763
(71) Anmelder: Beiersdorf AG, 20253 Hamburg (DE)
(72) Erfinder: Kröpke, Rainer, 22869 Schenefeld (DE); Von der Fecht, Stephanie, 22880 Wedel (DE); Treu, Jens, 22844 Norderstedt (DE); Schulz, Jens, 25462 Rellingen (DE)

(57) **Zusammenfassung**

Kosmetische Zubereitung enthaltend einen polymeren Verdicker sowie einen Bestandteil, welcher unter der CAS-No. 84082-60-0 registriert ist und/oder Lindenblütenextrakt.

## Beschreibung

Die vorliegende Erfindung betrifft eine Kombination aus polymerem Verdicker sowie CAS-No. 84082-60-0 bzw. Lindenblütenextrakt.

Polymere Verbindungen, beispielsweise Polyacrylate oder Polysaccharide, werden seit vielen Jahren als "Verdickungsmittel" zur Erhöhung der Viskosität insbesondere von wässrigen Lebensmitteln, kosmetischen Zubereitungen, Baustoffen, etc. eingesetzt. Eine besondere Klasse von polymeren Verdickungsmitteln stellen dabei die Polyacrylate dar.

Polyacrylate als Salze der Polyacrylsäure (meist Natrium oder Triethanolammoniumsalze) werden häufig zur Verdickung von kosmetischen Zubereitungen, insbesondere von Gelen auf Wasserbasis eingesetzt. Kosmetische Zubereitungen sind zum Teil hoch komplexe Mischungen (beispielsweise wässrige Gele oder Emulsionen) aus unterschiedlichsten chemischen Verbindungen. Ein Verdickungsmittel für kosmetische Zubereitungen muss daher eine hohe Verträglichkeit gegenüber einer Vielzahl von Substanzen aufweisen und eine hohe Temperaturstabilität, Lagerstabilität und eine hohe Toleranz gegenüber anderen Inhaltsstoffen aufweisen.

Soll die Zubereitung eine höhere, d.h. leicht zähfließende Viskosität aufweisen, müssen nach dem Stand der Technik relativ große Mengen an Polyacrylat der Zubereitung zugefügt werden, wodurch diese bei der Anwendung auf der Haut einen unangenehm schmierig-klebrigen Eindruck hinterlässt, welcher für den Anwender unakzeptabel ist.

Kosmetische O/W-Emulsionen enthalten üblicherweise 0,2 bis 1,0 Gewichts-% Polyacrylate damit die Zubereitung stabil (insbesondere langzeitstabil, d.h. lagerstabil) ist. In derartig hohen Einsatzkonzentrationen führen Polyacrylate dazu, dass sich die Zubereitung auf der Haut klebrig anfühlt und beim Verreiben der Zubereitung auf der Haut kleine "Röllchen" entstehen.

Es war daher die Aufgabe der vorliegenden Erfindung, die Nachteile des Standes der Technik zu beseitigen und viskose kosmetische Zubereitungen, insbesondere O/W-Emulsionen herzustellen, welche die Nachteile des Standes der Technik nicht mehr aufweisen.

Überraschend gelöst werden die Aufgaben durch eine kosmetische Zubereitung enthaltend einen polymeren Verdicker (insbesondere Polyacrylat (INCI Carbomer)) sowie einen Bestandteil, welcher unter der CAS-No. 84082-60-0 registriert ist und/oder Lindenblütenextrakt.

Überraschend gelöst werden die Aufgaben ferner durch die Verwendung des Bestandteils CAS-No. 84082-60-0 und/oder Lindenblütenextrakt zur Erhöhung der Viskosität von kosmetischen Zubereitungen enthaltend polymere Verdicker, insbesondere, wenn es sich bei dem polymeren Verdicker um Polyacrylat (INCI Carbomer) handelt.

Erfindungsgemäß ist auch eine kosmetische Zubereitung enthaltend 0,001 bis 1,0 Gewichts-% (bevorzugt 0,001 bis 0,19 Gewichts-%) Polyacrylat (INCI Carbomer) sowie einen Bestandteil, welcher unter der CAS-No. 84082-60-0 registriert ist und/oder Lindenblütenextrakt.

Überraschend gelöst werden die Aufgaben ferner durch die Verwendung des Bestandteils CAS-No. 84082-60-0 zur Erhöhung der Viskosität von kosmetischen Zubereitungen enthaltend Polyacrylat (INCI Carbomer) in einer Menge von 0,001 bis 0,19 Gewichts-% Polyacrylat, wobei sich die Gewichtsangabe auf das Gesamtgewicht der Zubereitung bezieht.

In der erfindungsgemäßen Zubereitung bzw. durch die erfindungsgemäße Verwendung kann die Viskosität der Zubereitung um mindestes 5% erhöht werden, so dass die Zubereitung im Bereich von +10°C bis -40°C eine geringere Viskosität aufweist, als ohne Parfumbestandteile..

Erfindungsgemäß vorteilhaft liegt die Viskosität der erfindungsgemäßen Zubereitung bei 100 bis 16.000mPas. Die erfindungsgemäße Viskosität wurde mit Hilfe eines Viscotester VT-02, Haake bei 25°C mit Hilfe des Drehkörpers 1 bzw. 2 und Ablesung der Skala 1 bzw. 2 bestimmt

Die erfindungsgemäßen Zubereitungen weisen ein angenehmes Hautgefühl auf. Die Neigung zur "Röllchenblidung" ist stark unterdrückt. Die Zubereitungen sind stabil und einfach und kostengünstig herstellbar.

Überraschend ist insbesondere, dass die Viskosität der Zubereitung bei sinkendem Carbomer-Gehalt ansteigt (siehe Vergleichsversuche).

Es ist erfindungsgemäß bevorzugt, wenn der Gehalt an Polyacrylat (INCI Carbomer) von 0,001 bis 1,0 Gewichts-% Polyacrylat, besonders bevorzug von 0,01 bis 0,5 Gewichts-% Polyacrylat und ganz besonders bevorzugt von 0,1 bis 0,25 Gewichts-% bezogen auf die Gesamtmenge der Zubereitung, beträgt.

Der Begriff "erfindungsgemäß" bezieht sich dabei im Rahmend dieser Offenbarung sowohl auf die erfindungsgemäße Zubereitung als auch auf die erfindungsgemäße Verwendung.

Es ist erfindungsgemäß vorteilhaft, wenn die Zubereitung in Form einer Emulsion vorliegt.

Es ist erfindungsgemäß bevorzugt, wenn die Zubereitung in Form einer O/W-Emulsion vorliegt.

Liegt die erfindungsgemäße Zubereitung in Form einer O/W-Emulsion vor, so ist sie erfindungsgemäß bevorzugt **dadurch gekennzeichnet, dass** die Zubereitung einen oder mehrere O/W-Emulgatoren gewählt aus der Gruppe der Verbindungen Glycerylstearatcitrat, Glycerylstearat (selbstemulgierend), Stearinsäure, Stearatsalze, Polyglyceryl-3-methylglycosedistearat, Ceteareth-20, PEG-40 Stearat , Natriumcetearylsulfat enthält. Ferner ist es vorteilhaft im Sinne der vorliegenden Erfindung Cetearylalkohol in Kombination mit PEG-40 hydriertes Rizinusöl, Natriumcetearylsulfat und Glycerylstearat. Ausserdem ist es erfindungsgemäß vorteilhaft Kaliumcetylphosphat als Emulgator einzusetzen.

Diese erfindungsgemäßen O/W-Emulgatoren können erfindungsgemäß vorteilhaft in einer Konzentration von 0,001 bis 10 Gewichts-% und bevorzugt in einer Konzentration von 0,1 bis 7 Gewichts-%, bezogen auf das Gesamtgewicht der Zubereitung in dieser enthalten sein.

Erfindungsgemäß vorteilhafte Ausführungsformen der vorliegenden Erfindung sind **dadurch gekennzeichnet, dass** die Zubereitung einen oder mehrere Parfümstoffe gewählt aus der Gruppe der Verbindungen Linaylacetat, Citral, Limonen, Linalool, Anisalkohol, Benzylalkohol, Benzylsalicylat, Cinnamylalkohol enthält.

Dabei ist es erfindungsgemäß von Vorteil, wenn die Zubereitung Parfümstoffe gewählt aus der Gruppe der Verbindungen Linaylacetat, Citral, Limonen, Linalool, Anisalkohol, Benzylalkohol, Benzylsalicylat, Cinnamylalkohol in einer Gesamtkonzentration von 0,0001 bis 10 Gewichts-%, bezogen auf das Gewicht des Parfums, enthält.

Es ist erfindungsgemäß vorteilhaft, wenn die Zubereitung den Bestandteil CAS-No. 84082-60-0 in einer Konzentration von 0,01 bis 10 Gewichts-%, bezogen auf das Gesamtgewicht der Zubereitung, enthält.

Es ist erfindungsgemäß bevorzugt, wenn die Zubereitung den Bestandteil CAS-No. 84082-60-0 in einer Konzentration 0,01 bis 10 Gewichts-% (bevorzugt von 0,1 bis 5 Gewichts-%), bezogen auf das Gesamtgewicht der Zubereitung, enthält.

Bei dem Bestandteil CAS-No. 84082-60-0 (EINECS No. 282-006-5) handelt es sich um einen Extrakt der Blüten der Chamomilla recutita (bevorzugt aus deutscher Herkunft), der mit der INCI Chamomilla Recutita Flower Extract oder Chamomilla Recutita Extract deklariert wird.

Es ist erfindungsgemäß vorteilhaft, wenn die Zubereitung den Bestandteil Lindenblütenextrakt in einer Konzentration von 0,01 bis 10 Gewichts-%, bezogen auf das Gesamtgewicht der Zubereitung, enthält.

Es ist erfindungsgemäß bevorzugt, wenn die Zubereitung den Bestandteil Lindenblütenextrakt in einer Konzentration von 0,1 bis 5 Gewichts-%, bezogen auf das Gesamtgewicht der Zubereitung, enthält.

Es ist erfindungsgemäß besonders bevorzugt, wenn die Zubereitung den Bestandteil Lindenblütenextrakt in einer Konzentration von 0,25 bis 1 Gewichts-%, bezogen auf das Gesamtgewicht der Zubereitung, enthält.

Bei dem Bestandteil Lindenblütenextrakt handelt es sich um einen Extrakt der Blüten der Linden. Der Extrakt ist unter *Tilia* in der INCI-Nomenklatur gelistet.

Die bevorzugten Blüten können von folgenden Lindenarten genommen werden:
*Amerikanische Linde (Tilia americana)*
*Tilia amurensis*
*Tilia caucasica*
*Tilia chenmoui*
*Tilia chinensis*
*Tilia chingiana*
*Winterlinde (Tilia cordata): Auch Steinlinde genannt; Syn. Tilia parvifolia*
*Krim-Linde (Tilia* × *euchlora*)
*Holländische Linde (Tilia* × *europaea) (Syn.: T.* × *intermedia, T.* × *vulgaris)*
*Kaiserlinde (Tilia* × *europaea 'Pallida')*
*Tilia 'Harold Hillier'*
*Tilia henryana*
*Tilia hupehensis*
*Tilia insularis*
*Tilia intonsa*
*Tilia japonica*
*Tilia kiusiana*
*Tilia koreana*
*Tilia laetevirens*
*Tilia ledbourii*
*Tilia mandshurica*
*Tilia maximowicziana*
*Tilia mexicana*
*Tilia miqueliana*
*Moltke-Linde (Tilia* × *moltkei)*
*Tilia mongolica*
*Olivers Linde (Tilia oliveri)*
*Tilia paucicostata*
*Hänge-Si*/*ber-Linde (Tilia petiolaris)*
*Sommerlinde (Tilia platyphyllos)*
*Tilia tarquetii*
*Silber-Linde (Tilia tomentosa)*
*Tilia tuan*
*Tilia* × *varsaviensis*

Es ist erfindungsgemäß von Vorteil, wenn die Zubereitung einen oder mehrere Konservierungsstoffe enthält, welche gewählt werden aus der Gruppe der Verbindungen Methylparaben, Ethylparaben, Propylparaben, Butylparaben, Phenoxyethanol.

Es ist erfindungsgemäß von Vorteil, wenn die Zubereitung frei von Verbindungen aus der Gruppe der Isobutylparaben, lodopropynylbutylcarbamat und Diazolidinylharnstoff ist.

Dabei ist es erfindungsgemäß bevorzugt, wenn die erfindungsgemäße Zubereitung Methylparaben, Ethylparaben, Propylparaben, Butylparaben, Phenoxyethanol in einer Gesamtmenge von 0,1 bis 2,5 Gewichts-%, bezogen auf das Gesamtgewicht der Zubereitung, enthält.

Es ist erfindungsgemäß vorteilhaft, wenn die Zubereitung einen oder mehrere Verbindungen gewählt aus der Gruppe der Verbindungen der UV-Filter, alpha-Liponsäure, Folsäure, Phytoen, D-Biotin, Coenzym Q10, Niacinamid, alpha-Glucosylrutin, Carnitin, Carnosin, natürliche und/oder synthetische Isoflavonoide, Flavonoide, Kreatin, Kreatinin, Taurin, β-Alanin, Tocopherylacetat, Dihydroxyaceton; 8-Hexadecen-1,16-dicarbonsäure, Glycerylglycose, (2-Hydroxyethyl)harnstoff, Vitamin E bzw. seine Derivate und/oder Licochalcon A enthält, wobei als UV-Filter alle für die Kosmetik zugelassenen UV-Filter eingesetzt werden können.

Es ist erfindungsgemäß von Vorteil, wenn die Zubereitung ein oder mehrere Diole gewählt aus der Gruppe der Verbindungen 2-Methyl-1,3-propandiol, Pentan-1,2-diol, Hexan-1,2-diol, Heptan-1,2-diol, Octan-1,2-diol, Nonan-1,2-diol, Decan-1,2-diol enthält.

Derartige Diole können erfindungsgemäß vorteilhaft in einer Konzentration von 0,1 bis 20 Gewichts-%, bezogen auf das Gesamtgewicht der Zubereitung, in dieser enthalten sein.

Die erfindungsgemäßen Zubereitungen können ferner vorteilhaft auch Selbstbräunungssubstanzen enthalten, wie beispielsweise Dihydroxyaceton und/oder Melaninderivate in Konzentrationen von 1 Gew.-% bis zu 10 Gew.-%, bezogen auf das Gesamtgewicht der Zubereitung.

Ferner können die erfindungsgemäßen Zubereitungen auch Repellentien zum Schutz vor Mücken, Zecken und Spinnen und dergleichen enthalten. Vorteilhaft sind z. B. N,N-Diethyl-3-methylbenzamid (Handelsbezeichnung: Meta-delphene, "DEET"), Dimethylphtalat (Handelsbezeichnung: Palatinol M, DMP), 1-Piperidincarbonsäure-2-(2-hydroxyethyl)-1-methylpropylester sowie insbesondere 3-(N-n-Butyl-N-acetyl-amino)-propionsäureethylester (unter dem Handelsnamen Insekt Repellent® 3535 bei der Fa. Merck erhältlich). Die Repellentien können sowohl einzeln als auch in Kombination eingesetzt werden.

Als Feuchthaltemittel (Moisturizer) werden Stoffe oder Stoffgemische bezeichnet, welche kosmetischen Zubereitungen die Eigenschaft verleihen, nach dem Auftragen bzw. Verteilen auf der Hautoberfläche die Feuchtigkeitsabgabe der Hornschicht (auch transepidermal water loss (TEWL) genannt) zu reduzieren und/oder die Hydratation der Hornschicht positiv zu beeinflussen.

Vorteilhafte Feuchthaltemittel (Moisturizer) im Sinne der vorliegenden Erfindung sind beispielsweise Glycerin, Milchsäure und/oder Lactate, insbesondere Natriumlactat, Butylenglykol, Propylenglykol, Biosaccaride Gum-1, Glycine Soja, Ethylhexyloxyglycerin, Pyrrolidoncarbonsäure und Harnstoff. Ferner ist es insbesondere von Vorteil, polymere Moisturizer aus der Gruppe der wasserlöslichen und/oder in Wasser quellbaren und/oder mit Hilfe von Wasser gelierbaren Polysaccharide zu verwenden. Insbesondere vorteilhaft sind beispielsweise Hyaluronsäure, Chitosan und/oder ein fucosereiches Polysaccharid, welches in den Chemical Abstracts unter der Registraturnummer 178463-23-5 abgelegt und z. B. unter der Bezeichnung Fucogel®1000 von der Gesellschaft SOLABIA S.A. erhältlich ist. Moisturizer können vorteilhaft auch als Antifaltenwirkstoffe zum Schutz vor Hautveränderungen, wie sie z. B. bei der Hautalterung auftreten, verwendet werden.

Es ist vorteilhaft im Sinne der vorliegenden Erfindung, wenn die erfindungsgemäße Zubereitung ein oder mehrere Feuchthaltemittel in einer Gesamtkonzentration von 0,1 bis 20 Gewichts-% und bevorzugt in einer Gesamtkonzentration von 0,5 bis 10 Gewichts-%, jeweils bezogen auf das Gesamtgewicht der Zubereitung, enthält.

Erfindungsgemäß vorteilhaft ist die erfindungsgemäße Zubereitung frei von Glucosylglyceriden (Glycerylglucose).

Die erfindungsgemäßen kosmetischen Zubereitungen können ferner vorteilhaft, wenngleich nicht zwingend, Füllstoffe enthalten, welche z. B. die sensorischen und kosmetischen Eigenschaften der Formulierungen weiter verbessern und beispielsweise ein samtiges oder seidiges Hautgefühl hervorrufen oder verstärken. Vorteilhafte Füllstoffe im Sinne der vorliegenden Erfindung sind Stärke und Stärkederivate (wie z. B. Tapiocastärke, Distärkephosphat, Aluminium- bzw. Natrium-Stärke Octenylsuccinat und dergleichen), Pigmente, die weder hauptsächlich UV-Filter- noch färbende Wirkung haben (wie z. B. Bornitrid etc.) und/oder Aerosile^{®} (CAS-Nr. 7631-86-9) und /oder Talkum.

Die Wasserphase der erfindungsgemäßen Zubereitungen kann vorteilhaft übliche kosmetische Hilfsstoffe enthalten, wie beispielsweise Alkohole, insbesondere solche niedriger C-Zahl, vorzugsweise Ethanol und/oder Isopropanol oder Polyole niedriger C-Zahl sowie deren Ether, vorzugsweise Propylenglykol, Glycerin, Ethylenglykol, Ethylenglykolmonoethyl- oder -monobutylether, Propylenglykolmonomethyl, -monoethyl- oder -monobutylether, Diethylenglykolmonomethyl- oder -monoethylether und analoge Produkte.

Die Ölphase der erfindungsgemäßen Zubereitung wird vorteilhaft gewählt aus der Gruppe der polaren Öle, beispielsweise aus der Gruppe der Lecithine und der Fettsäuretriglyceride, namentlich der Triglycerinester gesättigter und/oder ungesättigter, verzweigter und/oder unverzweigter Alkancarbonsäuren einer Kettenlänge von 8 bis 24, insbesondere 12 bis 18 C-Atomen. Die Fettsäuretriglyceride können beispielsweise vorteilhaft gewählt werden aus der Gruppe der synthetischen, halbsynthetischen und natürlichen Öle, wie z. B. Cocoglycerid, Olivenöl, Sonnenblumenöl, Jojobaöl, Sojaöl, Erdnußöl, Rapsöl, Mandelöl, Palmöl, Kokosöl, Rizinusöl, Weizenkeimöl, Traubenkernöl, Distelöl, Nachtkerzenöl, Macadamianußöl und dergleichen mehr.

Erfindungsgemäß vorteilhaft sind ferner z. B. natürliche Wachse tierischen und pflanzlichen Ursprungs, wie beispielsweise Bienenwachs und andere Insektenwachse sowie Beerenwachs, Sheabutter und/oder Lanolin (Wollwachs).

Weitere vorteilhafte polare Ölkomponenten können im Sinne der vorliegenden Erfindung ferner gewählt werden aus der Gruppe der Ester aus gesättigten und/oder ungesättigten, verzweigten und/oder unverzweigten Alkancarbonsäuren einer Kettenlänge von 3 bis 30 C-Atomen und gesättigten und/oder ungesättigten, verzweigten und/oder unverzweigten Alkoholen einer Kettenlänge von 3 bis 30 C-Atomen sowie aus der Gruppe der Ester aus aromatischen Carbonsäuren und gesättigten und/oder ungesättigten, verzweigten und/oder unverzweigten Alkoholen einer Kettenlänge von 3 bis 30 C-Atomen. Solche Esteröle können dann vorteilhaft gewählt werden aus der Gruppe Phenethylbenzoat, 2-Phenylethylbenzoat, Isopropyl Lauroyl Sarkosinat, Phenyl Trimethicon, Cyclomethicon, Dibutyladipat, Octylpalmitat, Octylcocoat, Octylisostearat, Octyldodeceylmyristat, Octyldodekanol, Cetearylisononanoat, Isopropylmyristat, Isopropylpalmitat, Isopropylstearat, Isopropyloleat, n-Butylstearat, n-Hexyllaurat, n-Decyloleat, Isooctylstearat, Isononylstearat, Isononylisononanoat, 2-Ethylhexylpalmitat, 2-Ethylhexyllaurat, 2-Hexyldecyl-stearat, 2-Octyldodecylpalmitat, Stearylheptanoat, Oleyloleat, Oleylerucat, Erucyloleat, Erucylerucat, Tridecylstearat, Tridecyltrimellitat, sowie synthetische, halbsynthetische und natürliche Gemische solcher Ester, wie z. B. Jojobaöl.

Ferner kann die Ölphase vorteilhaft gewählt werden aus der Gruppe der Dialkylether und Dialkylcarbonate, vorteilhaft sind z. B. Dicaprylylether (*Cetiol OE*) und/oder Dicaprylylcarbonat, beispielsweise das unter der Handelsbezeichnung *Cetiol* CC bei der Fa. Cognis erhältliche.

Es ist ferner bevorzugt, das oder die Ölkomponenten aus der Gruppe Isoeikosan, Neopentylglykoldiheptanoat, Propylenglykoldicaprylat/dicaprat, Caprylic/Capric/Diglycerylsuccinat, Butylenglykol Dicaprylat/Dicaprat, C₁₂₋₁₃-Alkyllactat, Di-C₁₂₋₁₃-Alkyltartrat, Triisostearin, Dipentaerythrityl Hexacaprylat/Hexacaprat, Propylenglykolmonoisostearat, Tricaprylin, Dimethylisosorbid. Es ist insbesondere vorteilhaft, wenn die Ölphase der erfindungsgemäßen Formulierungen einen Gehalt an C₁₂₋₁₅-Alkylbenzoat aufweist oder vollständig aus diesem besteht.

Vorteilhafte Ölkomponenten sind ferner z. B. Butyloctylsalicylat (beispielsweise das unter der Handelsbezeichnung *Hallbrite BHB* bei der Fa. CP Hall erhältliche), Tridecylsalicylat (welches unter der Handelsbezeichnung Cosmacol ESI bei der Fa. Sasol erhältlich ist), C12-C15 Alkylsalicylat (unter der Handelsbezeichnung Dermol NS bei der Fa. Alzo erhältlich), Hexadecylbenzoat und Butyloctylbenzoat und Gemische davon (*Hallstar AB)* und/oder Diethylhexylnaphthalat (*Hallbrite TQ oder Corapan TQ von Symrise).*

Auch beliebige Abmischungen solcher Öl- und Wachskomponenten sind vorteilhaft im Sinne der vorliegenden Erfindung einzusetzen.

Ferner kann die Ölphase ebenfalls vorteilhaft auch unpolare Öle enthalten, beispielsweise solche, welche gewählt werden aus der Gruppe der verzweigten und unverzweigten Kohlenwasserstoffe und -wachse, insbesondere Mineralöl, Vaseline (Petrolatum), Paraffinöl, Squalan und Squalen, Polyolefine, hydrogenierte Polyisobutene, C13-16 Isoparaffin und Isohexadecan. Unter den Polyolefinen sind Polydecene die bevorzugten Substanzen.

Es ist erfindungsgemäß bevorzugt, wenn die Zubereitung Flüssigparaffin enthält. Dieses kann erfindungsgemäß vorteilhaft ineiner Konzentration von bis Gewichts-%, bezogen auf das Gesamtgewicht der Zubereitung, eingesetzt werden.

Die erfindungsgemäßen Zubereitungen können ferner vorteilhaft eine oder mehrere Substanzen aus der folgenden Gruppe der Siloxanelastormere enthalten, beispielsweise um die Wasserfestigkeit und/oder den Lichtschutzfaktor der Produkte zu steigern:
(a) Siloxanelastomere, welche die Einheiten R₂SiO und RSiO_{1,5} und/oder R₃SiO_{0,5} und/oder SiO₂ enthalten,
   wobei die einzelnen Reste R jeweils unabhängig voneinander Wasserstoff, C₁₋₂₄-Alkyl (wie beispielsweise Methyl, Ethyl, Propyl) oder Aryl (wie beispielsweise Phenyl oder Tolyl), Alkenyl (wie beispielsweise Vinyl) bedeuten und das Gewichtsverhältnis der Einheiten R₂SiO zu RSiO_{1,5} aus dem Bereich von 1 : 1 bis 30 : 1 gewählt wird;
(b) Siloxanelastomere, welche in Silikonöl unlöslich und quellfähig sind, die durch die Additionsreaktion eines Organopolysiloxans (1), das siliciumbebundenen Wasserstoff enthält, mit einem Organopolysiloxan (2), das ungesättigte aliphatische Gruppen enthält, erhältlich sind,
wobei die verwendeten Mengenateile so gewählt werden, dass die Menge des Wasserstoffes des Organopolysiloxans (1) oder der ungesättigten aliphatischen Gruppen des Organopolysiloxans (2)
- im Bereich von 1 bis 20 mol-% liegt, wenn das Organopolysiloxan nicht zyklisch ist und
- im Bereich von 1 bis 50 mol-% liegt, wenn das Organopolysiloxan zyklisch ist.

Vorteilhaft im Sinne der vorliegenden Erfindung liegen das oder die Siloxanelastomere in Form sphärischer Puder oder in Form von Gelen vor.

Erfindungsgemäß vorteilhafte in Form sphärischer Puder vorliegende Siloxanelastomere sind die mit der INCI-Bezeichnung Dimethicone / Vinyl Dimethicone Crosspolymer, beispielsweise das von DOW CORNING unter der Handelsbezeichnungen DOW CORNING 9506 Powder erhältliche.

Besonders bevorzugt ist es, wenn das Siloxanelastomer in Kombination mit Ölen aus Kohlenwasserstoffen tierischer und/oder pflanzlicher Herkunft, synthetischen Ölen, synthetischen Estern, synthetischen Ethern oder deren Gemischen verwendet wird.

Besonders vorteilhafte Zubereitungen werden ferner erhalten, wenn als Zusatz- oder Wirkstoffe Antioxidantien eingesetzt werden. Erfindungsgemäß enthalten die Zubereitungen vorteilhaft eines oder mehrere Antioxidantien. Als günstige, aber dennoch fakultativ zu verwendende Antioxidantien können alle für kosmetische Anwendungen geeigneten oder gebräuchlichen Antioxidantien verwendet werden.

Besonders vorteilhaft im Sinne der vorliegenden Erfindung können wasserlösliche Antioxidantien eingesetzt werden, wie beispielsweise Vitamine, z. B. Ascorbinsäure und deren Derivate.

Bevorzugte Antioxidantien sind ferner Vitamin E und dessen Derivate sowie Vitamin A und dessen Derivate.

Die Menge der Antioxidantien (eine oder mehrere Verbindungen) in den Zubereitungen beträgt vorzugsweise 0,001 bis 30 Gew.-%, besonders bevorzugt 0,05 bis 20 Gew.-%, insbesondere 0,1 bis 10 Gew.-%, bezogen auf das Gesamtgewicht der Zubereitung.

Besonders vorteilhaft im Sinne der vorliegenden Erfindung können Zusatzstoffe eingesetzt werden, die die Langzeitstabilität der Zubereitung verbessern..

### Bevorzugte Stabilitätsverbesserer sind:

Acetyltrifluoromethylphenylvalylglycin, Acrylamidammoniumacrylatcopolymer, Aluminummagnesiumhydroxidstearat, Ammoniumlactat, Ammoniumpolyacrylat, Ammoniumpolyacryloyldimethyltaurat, Arginin PCA, Capryloylsalicylsäureester, Zimtsäure, Cocoglucosid, Kupfergluconat, Diphenyldimethicon, Dinatriumadenosintriphosphat, Dnatriumsuccinat, Disteardimoniumhectorit, Dodecen, Eperua Falcata , hydriertes Palmenglycerid, hydriertes Palmenglyceridcitrat, Hydrierte Palmenkernglyceride, Hydrolisiertes Weizenprotein PG-Propylmethylsilandiol, Hydroxyethylacrylat / Natriumacryloyldimethyltauracopolymer, Isodeceth-6, Linseed Acid Magnesiumaspartat, Melibiose, Oxothiazolidinecarboxylsäure, Palmitoylpentapeptide 4, PEG-8 Laurat, Phenethylalkohol, Phenylpropanol, Polyacrylate-13, Polyacrylate-3 , Sarcosin, Saxifraga Sarmentosa Extrakt, Scutellaria Baicalensis Extrakt , Natriummetabisulfit, Sojaisoflavon, Tocopherylglucosid, Trideceth-6, Zinkgluconat.

Erfindungsgemäß bevorzugt ist die erfindungsgemäße Verwendung, die dadurch gekennzeichnet ist, dass die Zubereitung im Bereich von +10°C bis -40°C eine geringere Viskosität aufweist, als ohne Parfumbestandteile.

### Vergleichsversuche

Die folgenden Experimente können die erfindungsgemäßen Eigenschaften der vorliegenden Erfindung verdeutlichen (ohne dass die Erfindung darauf beschränkt wäre):

**Rezepturen:**

| **Bestandteil** | **Rezeptur 1 "Gonlo 5"** | **Rezeptur 1 "Gonlo 6"** | **Rezeptur 1 "Gonlo 7** |
|---|---|---|---|
| Methylparaben | 0,3 | 0,3 | 0,3 |
| med. Weissöl | 10 | 10 | 10 |
| Glycerylstearat | 1,5 | 1,5 | 1,5 |
| Cetylalkohol | 2,25 | 2,25 | 2,25 |
| PEG-40 Stearat | 0,75 | 0,75 | 0,75 |
| Phenoxyethanol | 0,75 | 0,75 | 0,75 |
| Glycerin | 3 | 3 | 3 |
| Natriumcarbomer | 0,02 | 0,1 | 0,175 |
| Parfum | 0,2 | 0,2 | 0,2 |
| Kamillenextrakt | 0,25 | 0,25 | 0,25 |

**Viskositätswerte:**

| **Viskosität** | **GONLO_5** | **GONLO_6** | **GONLO_7** |
|---|---|---|---|
| nach 1 Tag | 7950,0000 mPas | 6300,0000 mPas | 4500,0000 mPas |
| nach 7 Tagen | 7800,0000 mPas | 6000,0000 mPas | 4350,0000 mPas |
| nach 14 Tagen | 7700,0000 mPas | 5350,0000 mPas | 4600,0000 mPas |
| nach 28 Tagen | 6350,0000 mPas | 5350,0000 mPas | 4350,0000 mPas |
| nach 90 Tagen | 6600,0000 mPas | 4500,0000 mPas | 4050,0000 mPas |
| nach 180 Tagen | 6450,0000 mPas | 4400,0000 mPas | 3850,0000 mPas |

### Beispiele

Die nachfolgenden Beispiele sollen die vorliegende Erfindung verdeutlichen, ohne sie einzuschränken. Alle Mengenangaben, Anteile und Prozentanteile sind, soweit nicht anders angegeben, auf das Gewicht und die Gesamtmenge bzw. auf das Gesamtgewicht der Zubereitungen bezogen.

**O/W-Emulsion**

| | **1** | **2** | **3** | **4** | **5** |
|---|---|---|---|---|---|
| Glycerylsterat | 1,0 | --- | -- | 0,5 | 0,25 |
| Polyethylenglycol(40)stearat | 10,0 | --- | 5 | -- | -- |
| Triglycerinmethylglucosedistearat | --- | 5,5 | --- | --- | 2,5 |
| Sorbitanstearat | --- | 1,5 | 3 | --- | --- |
| Cyclomethicon | 2,5 | 15 | 8,0 | 5,0 | 7,5 |
| Dimethicon | 5,0 | 3,0 | 5,0 | 2,0 | 15,0 |
| Behenylalkohol | 1 | --- | 2 | 1 | --- |
| Stearylalkohol | --- | 1 | --- | 1 | --- |
| Cetylstearylalkohol | --- | --- | 1 | 1 | --- |
| hydriertes Polyisobuten | 0,5 | 0,75 | 1,0 | 2,0 | 0,25 |
| Octyldodecanol | 0,5 | 1,0 | 0,75 | 3,0 | 0,25 |
| Lindenblütenextrakt | 5 | 1,5 | 0,75 | 0,35 | 0,1 |
| Parfum | 0,25 | 0,1 | 0,35 | 0,45 | 1,0 |
| Linalylacetate* | 0,025 | 0,001 | 0,0001 | 0,045 | 0,0025 |
| Methylparaben | 0,4 | 0,1 | 0,05 | 0,3 | 0,4 |
| Propylparaben | 0,3 | 0,4 | 0,25 | 0,15 | --- |
| Benzylalkohol | --- | --- | 0,05 | --- | 0,1 |
| Glycerin | 5 | 10 | 3 | 15 | 7,5 |
| Kamillenextrakt | 0,1 | 2,5 | 0,75 | 0,15 | 1,5 |
| Wasser | ad 100 | ad 100 | ad 100 | ad 100 | ad 100 |

| | | | | | |
|---|---|---|---|---|---|
| *bezogen auf das Gewicht des Parfumöls | | | | | |

**O/W-Emulsion**

| | **6** | **7** | **8** | **9** | **10** |
|---|---|---|---|---|---|
| Polyethylenglycol(40)stearat | 0,75 | 1,5 | 0,75 | 0,9 | 1,5 |
| Glycerylstearat | 1,5 | 1,5 | 1,5 | 1,8 | 2,5 |
| Cetylalkohol | 2,25 | 1,5 | 0,75 | 2,7 | 0,75 |
| medizinisches Weissöl | 10 | 15 | 10 | 10 | 10 |
| Phenoxyethanol | 0,2 | 0,1 | 0,2 | 0,2 | 0,35 |
| Kamillenextrakt | 0,25 | 0,25 | 0,25 | 0,25 | 1,0 |
| Lindenblütenextrakt | 0,1 | 0,1 | 0,25 | 3,5 | 1,0 |
| Glycerin 99% | 3,5 | 3,5 | 3,75 | 3,8 | 22,5 |
| Natriumcarbomer | 0,02 | 0,4 | 0,2 | 0,02 | 0,1 |
| Parfum | 0,25 | 0,5 | 0,25 | 0,25 | 0,85 |
| Methylparaben | 0,2 | 0,1 | 0,2 | 0,2 | 0,4 |
| Citral* | 0,037 | 0,02 | 0,037 | 0,001 | 0,1 |
| Cinamylalkohol* | 0,002 | 0,02 | 0,002 | 0,001 | 0,1 |
| Anisalkohol* | 0,0038 | 0,02 | 0,1 | 0,0038 | 0,0076 |
| Wasser | ad 100 | ad 100 | ad 100 | ad 100 | ad 100 |

| | | | | | |
|---|---|---|---|---|---|
| *bezogen auf das Gewicht des Parfumöls | | | | | |

## Patentansprüche

1. Kosmetische Zubereitung enthaltend einen polymeren Verdicker sowie einen Bestandteil, welcher unter der CAS-No. 84082-60-0 registriert ist und/oder Lindenblütenextrakt.

2. Verwendung des Bestandteils CAS-No. 84082-60-0 und/oder Lindenblütenextrakt zur Erhöhung der Viskosität von kosmetischen Zubereitungen enthaltend polymere Verdicker.

3. Kosmetische Zubereitung enthaltend 0,001 bis 1,0 Gewichts-% Polyacrylat (INCI Carbomer) sowie einen Bestandteil, welcher unter der CAS-No. 84082-60-0 registriert ist und/oder Lindenblütenextrakt.
wobei sich die Gewichtsangabe auf das Gesamtgewicht der Zubereitung bezieht.

4. Kosmetische Zubereitung oder Verwendung nach einem der vorhergehenden Ansprüche, **dadurch gekennzeichnet, dass** die Zubereitung in Form einer O/W-Emulsion vorliegt.

5. Kosmetische Zubereitung oder Verwendung nach einem der vorhergehenden Ansprüche, **dadurch gekennzeichnet, dass** die Zubereitung einen oder mehrere Parfümstoffe gewählt aus der Gruppe der Verbindungen Linaylacetat, Citral, Limonene, Linalool, Anisalkohol, Benzylalkohol, Benzylsalicylat, Cinnamylalkohol enthält.

6. Kosmetische Zubereitung oder Verwendung nach einem der vorhergehenden Ansprüche, **dadurch gekennzeichnet, dass** die Zubereitung den Bestandteil CAS-No. 84082-60-0 in einer Konzentration von 0,01 bis 10 Gewichts-%, bezogen auf das Gesamtgewicht der Zubereitung, enthält.

7. Kosmetische Zubereitung oder Verwendung nach einem der vorhergehenden Ansprüche, **dadurch gekennzeichnet, dass** die Zubereitung einen oder mehrere O/W-Emulgatoren gewählt aus der Gruppe der Verbindungen Glycerylstearatcitrat, Glycerylstearat (selbstemulgierend), Stearinsäure, Stearatsalze, Polyglyceryl-3-methylglycosedistearat, Ceteareth-20, PEG-40 Stearat, PEG-100 Stearat, Sucrose Polystearat in Kombination mit hydriertem Polyisobuten, Natriumstearoylglutamat, Natriumcetearylsulfat enthält.

8. Kosmetische Zubereitung oder Verwendung nach einem der vorhergehenden Ansprüche, **dadurch gekennzeichnet, dass** die Zubereitung einen oder mehrere Konservierungsstoffe enthält, welche gewählt werden aus der Gruppe der Verbindungen Methylparaben, Ethylparaben, Propylparaben, Butylparaben und Phenoxyethanol.

9. Kosmetische Zubereitung oder Verwendung nach einem der vorhergehenden Ansprüche, **dadurch gekennzeichnet, dass** die Zubereitung Flüssigparaffin enthält.

10. Verwendung nach einem der vorhergehenden Ansprüche, **dadurch gekennzeichnet, dass** die Viskosität der Zubereitung um mindestes 5 % erhöht wird.

11. Verwendung nach einem der vorhergehenden Ansprüche, **dadurch gekennzeichnet, dass** die Zubereitung im Bereich von +10°C bis -40°C eine geringere Viskosität aufweist, als ohne Parfumbestandteile.
